Europäisches Patentamt

European Patent Office

Office européen des brevets

(11) Veröffentlichungsnummer: **0 218 051**
A1

(12) # EUROPÄISCHE PATENTANMELDUNG

(21) Anmeldenummer: 86111337.1

(22) Anmeldetag: 16.08.86

(51) Int. Cl.⁴: **A 23 K 1/175**, A 61 K 9/22, A 61 K 47/00

(30) Priorität: 11.09.85 DE 3532363

(43) Veröffentlichungstag der Anmeldung: 15.04.87
Patentblatt 87/16

(84) Benannte Vertragsstaaten: AT BE CH DE FR GB IT LI LU NL SE

(71) Anmelder: **Busch, Manfred, Hohkeppeler Strasse 63, D-5061 Heiligenhaus (DE)**

(72) Erfinder: **Busch, Manfred, Hohkeppeler Strasse 63, D-5061 Heiligenhaus (DE)**

(74) Vertreter: **Lippert, Hans-Joachim, Dipl.-Ing. et al, Dipl.-Ing. W. Dahlke Dipl.-Ing. H.-J. Lippert Patentanwälte Frankenforster Strasse 137, D-5060 Bergisch Gladbach 3 (DE)**

(54) **Heilmittel für Pferde.**

(57) Heilmittel für Pferde bestehen beispielsweise aus pharmazeutischen Präparaten und/oder Wirkstoffen von Heilpflanzen, die mit einem genießbaren Bindemittel zu einer bei normalen Umgebungstemperaturen festen Substanz vermischt und zu einem Leckstein geformt sind. Um das Heilmittel derart weiterzubilden, daß es problemlos verabreicht werden kann und den Pferden gut bekommt, enthält die zu dem Leckstein geformte Substanz als Bindemittel ein wohlschmeckendes Gemisch aus Zucker, Glukose, Honig und Salz. Eine solche Substanz wird von allen Pferden gern angenommen und kann ohne schädigende Wirkungen auch in größeren Mengen verabreicht werden. Eine Vorrichtung zur Verabreichung des Lecksteins besteht aus mindestens einem unten offenen Behälter (5), in welchem der Leckstein (1) mit Hilfe eines Halteelements (2) derart befestigbar ist, daß er ein Stück aus der unteren Öffnung des Behälters herausragt.

ACTORUM AG

Dipl.-Ing. W. Dahlke
Dipl.-Ing. H.-J. Lippert
Patentanwälte
Frankenforster Straße 137
5060 Bergisch Gladbach 1

0218051

⁄ -1-

15. August 1986
L/Sti

Manfred Busch
5061 Heiligenhaus

## "Heilmittel für Pferde"

Die Erfindung betrifft ein Heilmittel für Pferde, bestehend aus pharmazeutischen Präparaten und/oder Wirkstoffen von Heilpflanzen, die mit einem genießbaren Bindemittel zu einer bei normalen Umgebungstemperaturen festen Substanz vermischt und zu einem Leckstein geformt sind.

Heilmittel für Pferde werden normalerweise in Form von Säften und Pulvern im Handel angeboten. Zur Verabreichung werden Sie dem Futter oder dem Trinkwasser der Pferde beigemischt. Diese Form der Verabreichung ist für das Pflegepersonal arbeitsaufwendig, da das Heilmittel jeweils dosiert und bei jedem einzelnen kranken Pferd dem Futter oder Wasser beigemischt werden muß. Darüber hinaus besteht bei dieser Verabreichungsform der Nachteil, daß das Futter und Wasser eine leichte Geschmacksveränderung erfahren kann und deshalb von den Pferden nicht angenommen wird.

Es ist deshalb bereits versucht worden, bestimmte Arzneimittel Salzlecksteinen beizumischen (DE-OS 26 49 133). Diese Methode hat jedoch den Nachteil, daß die Pferde, wenn ihnen bestimmte Arzneimittelmengen verabreicht werden sollen, relativ große Salzmengen zu sich nehmen müssen. Die Tiere werden sogar durch Beimengung von organoleptischen Substanzen zur Aufnahme des Salzes angeregt. Diese Methode hat sich jedoch nicht bewährt.

Der Erfindung liegt die Aufgabe zugrunde, ein Heilmittel für Pferde zu schaffen, das problemlos verabreicht werden kann und den Pferden gut bekommt.

Erfindungsgemäß wird diese Aufgabe dadurch gelöst, daß die zu einem Leckstein geformte Substanz als Bindemittel ein wohlschmeckendes Gemisch aus Zucker, Glukose, Honig und Salz enthält.

Eine solche Substanz wird von allen Pferden gern angenommen und kann ohne schädigende Wirkungen auch in größeren Mengen verabreicht werden.

Vorzugsweise enthält die Substanz ca. 90 Gewichtsprozent des Bindemittels und ca. 10 Gewichtsprozent an Heilpflanzenwirkstoffen. Bei einem solchen Mischungsverhältnis werden für das Pferd unangenehme Geschmacks- und Geruchsstoffe überdeckt und werden zusammen mit dem Leckstein problemlos aufgenommen. Ein Bindemittel aus ca. 57 % Zucker, 29 % Glukose, 4 % Honig und etwas weniger als 1 % Salz hat sich als besonders attraktiv für die Pferde erwiesen.

Bei einem Leckstein zur Bekämpfung von Husten enthält die Substanz vorzugsweise Wirkstoffe folgender Heilpflanzen: Eibisch, Efeu, Fenchel, Huflattich, Schlüsselblume, Spitzwegerich, Thymian, Stiefmütterchen, Lungenkraut, Salbei, Malve, Lindenblüten, Anis, Menthol und Holunder.

Eine Vorrichtung zur Verabreichung des in Form eines Lecksteins vorliegenden Heilmittels ist gekennzeichnet durch mindestens einen unten offenen Behälter, in welchem der Leckstein mit Hife eines Halteelements derart befestigbar ist, daß er ein Stück aus der unteren Öffnung herausragt. Durch diese Vorrichtung wird sichergestellt, daß das Pferd eine bestimmte Menge des Lecksteins zu sich nimmt, nämlich nur soviel, wie es mit der Zunge erreichen kann. Dadurch kann eine genaue Dosierung vorgenommen werden, indem der Leckstein in der Vorrichtung höher oder tiefer gehängt wird. In jedem Falle wird durch die Vorrichtung verhindert, daß das Pferd unter Umständen eine Überdosis des in dem Leckstein enthaltenen Arzneimittels zu sich nimmt, denn das Pferd kommt allenfalls an den aus der unteren Behälteröffnung herausragenden Abschnitt des Lecksteins heran.

Zweckmäßig weist der Leckstein eine längliche, im wesentlichen zylindrische Gestalt auf, wobei das Halteelement axial zu dem zylindrischen Leckstein angeordnet ist und sich ein kurzes Stück in das obere Stirnende des Lecksteins hineinerstreckt. Der Behälter der Vorrichtung kann für einen solchen Leckstein ein in vertikaler Anordnung befestigbares Rohr sein, dessen Innendurchmesser größer als der des Lecksteins ist.

Das Halteelement kann langgestreckt und flexibel und in den Leckstein eingegossen sein. Beispielsweise ist es als Kette aus Metallgliedern ausgebildet.

Um auf einfache Weise eine Höhenverstellung des Lecksteins zu erreichen, kann das Rohr in seinem oberen Rand einen Schlitz aufweisen, in den die in den Leckstein eingegossene Kette einhängbar ist, wobei jedes einzelne Kettenglied eingehängt und somit eine sehr feinstufige und unkomplizierte Verstellung des Lecksteins erzielt werden kann. Wenn das untere Ende des Lecksteins aufgebraucht ist, wird die Kette einfach aus dem Schlitz herausgehoben und dann mit dem folgenden Glied oder dem nächsten oder übernächsten Glied neu eingehängt, so daß der Leckstein nunmehr um die Länge eines oder mehrerer Kettenglieder tiefer hängt.

Als Rohr kann ein Abschnitt eines handelsüblichen Kunststoffrohrs verwendet werden, welches sich einfach verarbeiten läßt und im Gebrauch sehr wartungsfreundlich ist, da es leicht gereinigt werden kann und nicht korrodiert.

Zur Befestigung kann das Rohr an seinem unteren Ende und in einem Abstand darüber von je einer Schelle umgeben sein, während an den Schellen eine Halterung sitzt, mit der das Rohr an einer Wand der Box oder des Standes bzw. an einer an der Vorderfront der Box vorgesehenen vertikalen Stange befestigbar ist. Ein solcher Befestigungsmechanismus läßt sich jeweils an die gegebenen Örtlichkeiten anpassen und leicht anbringen. Insbesondere die an dem unteren Ende des Rohrs vorgesehene Schelle, die vorzugsweise aus Metall besteht und den unteren Rand des Rohrs überdeckt, verhindert, daß das Pferd den unteren Rand des Rohrs anknabbert.

Die Erfindung ist in der Zeichnung beispielsweise veranschaulicht und im nachstehenden im einzelnen anhand der Zeichnung beschrieben. Es zeigen:

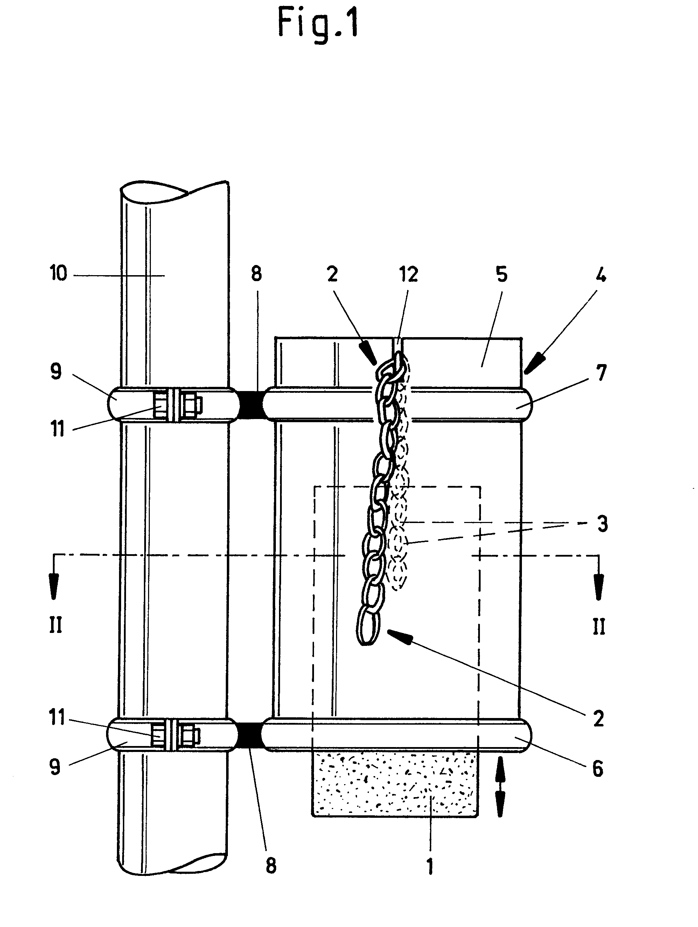

Fig. 1 ein Ausführungsbeispiel eines Lecksteins in einer Dosiervorrichtung und

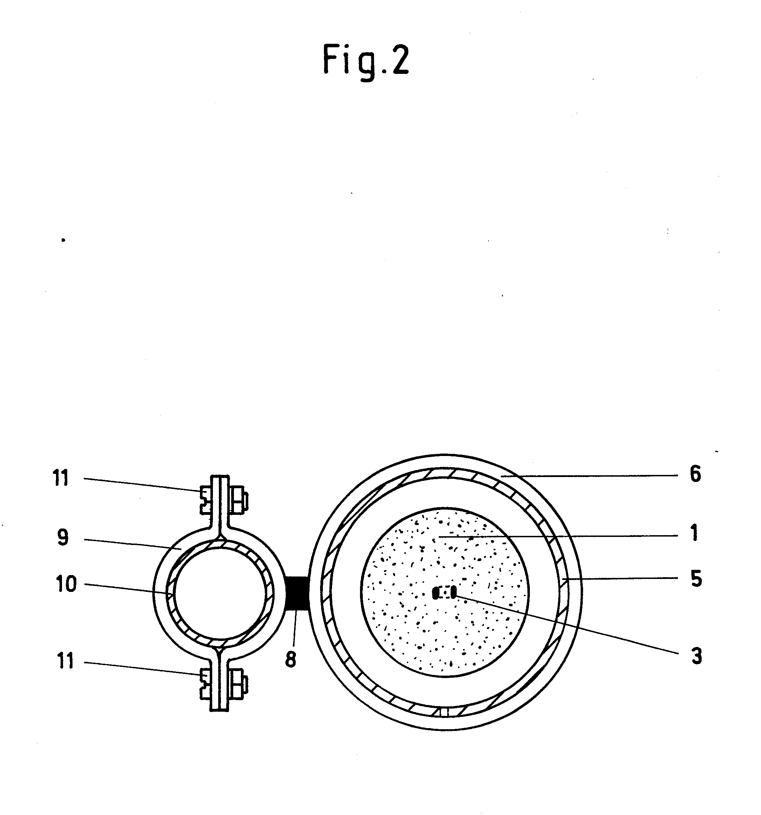

Fig. 2 einen Schnitt entlang der Linie II-II aus Fig. 1.

Der in der Zeichnung dargestellte Leckstein 1 besteht aus einer Mischung von verschiedenen Heilpflanzen, mit denen der Husten eines Pferdes bekämpft werden kann. Diese Heilpflanzenmischung ist fest in ein Bindemittel eingebunden, welches aus Zucker, Glukose, Honig und Salz besteht. Bei Raumtemperatur weist der Leckstein eine feste Konsistenz auf, so daß der Leckstein unter normalen klimatischen Bedingungen, die in einem Pferdestall herrschen, längere Zeit haltbar ist, ohne seine äußere Form zu verändern.

Der in der Zeichnung dargestellte Leckstein 1 weist eine längliche, zylindrische Gestalt auf. In ein Ende des Lecksteins 1 ist das Endstück einer Kette 2 eingegossen. Das eingegossene Ende besteht bei der in der Zeichnung dargestellten Ausführungsform aus etwa vier Kettengliedern 3, die ungefähr in der Längsachse des zylindrischen Lecksteins 1 liegen und von diesem fest umschlossen sind. Wenn daher das dem Leckstein abgewandte Ende der Kette 2 an einem Fixpunkt befestigt wird, hängt der Leckstein mit vertikal angeordneter Längsachse senkrecht nach unten.

Um zu verhindern, daß das Pferd in den Leckstein hineinbeißt und somit eine Überdosis des Hustenmittels zu sich nimmt, wird der Leckstein 1 nur in Verbindung mit einer Dosiervorrichtung 4 verabreicht.

Die Dosiervorrichtung 4 besteht aus einem Abschnitt eines handelsüblichen Kunststoffrohrs 5, dessen Innendurchmesser größer ist als der Außendurchmesser des Lecksteins 1, so daß der Leckstein 1 in das Rohr 5 hineinpaßt.

Das Kunststoffrohr 5 wird in vertikaler Anordnung an einer beliebigen Stelle in einer Box oder einem Stand befestigt. Bei dem in der Zeichnung dargestellten Ausführungsbeispiel ist das Rohr 5 an seinem unteren Ende und in einem Abstand von seinem oberen Ende von je einer Schelle 6 bzw. 7 aus nichtrostendem Metall fest umgeben. Die untere Schelle 6 sitzt genau in der Ebene des unteren Randes des Rohrs 5, so daß der untere Rand des Rohrs für das Pferd nicht zugänglich ist und beispielsweise nicht angeknabbert werden kann. Die beiden Schellen 6 und 7 sind über kurze Verbindungsstege 8 mit je einer geteilten Halteklammer 9 verbunden, die beispielsweise um eine vertikale Stange 10 herumgelegt und mit Hilfe von Schrauben 11 an dieser befestigt werden können.

Anstelle der Halteklammern 9 können alternativ auch einfache flache Laschen vorgesehen sein, die beispielsweise an der Wand der Box angeschraubt werden können.

In dem oberen Rand des Kunststoffrohrs 5 oberhalb der Schelle 7 ist ein kurzer vertikaler Schlitz 12 vorgesehen, der etwas breiter ist als die Dicke eines Kettengliedes 3 der in den Leckstein 1 eingegossenen Kette 2. Die Länge des Schlitzes ist ein Stück größer als die Breite eines Kettengliedes 3, so daß die Kette 2 bequem und sicher in den Schlitz 12 eingehängt werden kann.

Beim Einhängen eines frischen Lecksteins 1 wird dieser in dem Kunststoffrohr so positioniert, daß nur ein kurzes Stück des Lecksteins 1 aus dem unteren Ende des Rohrs 5 herausragt. In dieser Position ist nur dieses untere Ende des Lecksteins 1 für das Pferd zugänglich, so daß nur dieses Ende von dem Pferd aufgenommen werden kann, und zwar durch einfaches Anlecken, wodurch gleichzeitig erreicht wird, daß das Hustenmittel über einen längeren Zeitraum verteilt allmählich von dem Pferd aufgenommen werden kann.

Wenn das anfangs unten aus dem Rohr 5 herausragende Ende des Lecksteins 1 aufgebraucht ist, kann der Leckstein bei Bedarf neu positioniert werden, so daß der Leckstein wieder aus dem unteren Ende des Rohrs 5 ein Stück herausragt. Das neue Positionieren des Lecksteins 1 erfolgt einfach dadurch, daß die Kette 2 aus dem Schlitz 12 herausgehoben und daß ein anderen Kettenglied 3, welches sich in einem größeren Abstand von dem Leckstein 1 befindet, nunmehr in den Schlitz 12 eingeschoben wird.

Das Nachstellen des Lecksteins 1 kann so lange fortgesetzt werden, bis er völlig aufgebraucht ist. Zu diesem Zweck sind die Länge des Lecksteins 1, die Länge der Kette 2 und die Höhe des Kunststoffrohrs 5 aufeinander abgestimmt. Der Leckstein 1 ist höchstens so lang ausgebildet wie das Kunststoffrohr 5, vorzugsweise jedoch etwas kürzer, so daß er beim Einhängen in seiner obersten Position nur ein kurzes Stück aus dem Rohr 5 herausragt. Die Länge der Kette 2 ist so gewählt, daß beim Einhängen des vorletzten Kettengliedes das letzte, noch nicht aufgebrauchte Ende des Lecksteins 1 unten aus dem Rohr 5 herausragt, so daß der Leckstein in seiner tiefsten Position von dem zu behandelnden Pferd restlos aufgenommen werden kann.

Die beschriebene Vorrichtung kann selbstverständlich auch zur Verabreichung von anderen Lecksteinen verwendet werden, die eine andere Zusammensetzung oder eine von einem Zylinder abweichende Form aufweisen.

Dipl.-Ing. W. Dahlke
Dipl.-Ing. H.-J. Lippert
Patentanwälte
Frankenforster Straße 137
5060 Bergisch Gladbach 1

1

15. August 1986
L/Sti

**Manfred Busch**

**5061 Heiligenhaus**

## A n s p r ü c h e

1. Heilmittel für Pferde, bestehend aus pharmazeutischen Präparaten und/oder Wirkstoffen von Heilpflanzen, die mit einem genießbaren Bindemittel zu einer bei normalen Umgebungstemperaturen festen Substanz vermischt und zu einem Leckstein geformt sind, d a d u r c h  g e k e n n z e i c h n e t, daß die Substanz als Bindemittel ein wohlschmeckendes Gemisch aus Zucker, Glukose, Honig und Salz enthält.

2. Heilmittel für Pferde nach Anspruch 1, d a d u r c h  g e k e n n z e i c h n e t, daß die Substanz ca. 90 Gewichtsprozent Bindemittel enthält und ca. 10 Gewichtsprozent Heilpflanzenwirkstoffe.

3. Heilmittel für Pferde nach Anspruch 1 oder 2 zur Bekämpfung von Husten, d a d u r c h  g e k e n n z e i c h n e t, daß die Substanz Wirkstoffe folgender Heilpflanzen enthält: Eibisch, Efeu, Fenchel, Huflattich, Schlüsselblume, Spitzwegerich, Thymian, Stiefmütterchen, Lungenkraut, Salbei, Malve, Lindenblüten, Anis, Menthol und Holunder.

4. Vorrichtung zur Verabreichung des in Form eines Lecksteines vorliegenden Heilmittels nach einem der Ansprüche 1 bis 3, g e k e n n z e i c h n e t d u r c h mindestens einen unten offenen Behälter (5), in welchem der Leckstein (1) mit Hilfe eines Halteelementes (2) derart befestigbar ist, daß er ein Stück aus der unteren Öffnung des Behälters (5) herausragt.

5. Vorrichtung nach Anspruch 4, d a d u r c h g e - k e n n z e i c h n e t , daß der Leckstein (1) eine längliche, im wesentlichen zylindrische Gestalt aufweist, daß das Halteelement (2) axial zu dem zylindrischen Leckstein (1) angeordnet ist und sich ein kurzes Stück in das obere Stirnende des Lecksteins (1) hineinerstreckt und daß der Behälter ein in vertikaler Anordnung befestigbares Rohr (5) ist, dessen Innendurchmesser größer als der des Lecksteins (1) ist.

6. Vorrichtung nach Anspruch 4 oder 5, d a d u r c h g e k e n n z e i c h n e t , daß das Halteelement (2) langgestreckt und flexibel und in den Leckstein (1) eingegossen ist.

7. Vorrichtung nach Anspruch 6, d a d u r c h g e - k e n n z e i c h n e t , daß das Halteelement eine Kette (2) aus Metallgliedern (3) ist.

8. Vorrichtung nach Anspruch 7, d a d u r c h g e - k e n n z e i c h n e t , daß das Rohr in seinem oberen Rand einen Schlitz (12) aufweist, in den die in den Leckstein (1) eingegossene Kette (2) einhängbar ist.

9. Vorrichtung nach einem der Ansprüche 5 bis 8, d a d u r c h   g e k e n n z e i c h n e t ,   daß das Rohr (5) ein Abschnitt eines handelsüblichen Kunststoffrohrs ist.

10. Vorrichtung nach einem der Ansprüche 5 bis 9, d a d u r c h   g e k e n n z e i c h n e t ,   daß das Rohr (5) an seinem unteren Ende und in einem Abstand darüber von je einer Schelle (6, 7) umgeben ist und daß an den Schellen (6, 7) eine Halterung (9) sitzt, mit der das Rohr (5) an einer Wand bzw. einer vertikalen Stange (10) befestigbar ist.

Fig.1

0218051

1/2

Manfred Busch

# Fig.2

**Europäisches Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE-A-2 649 133 (P. NIGAY)<br>* Anspruch 1, Seite 8, Absatz 3 * | 1,2 | A 23 K 1/175<br>A 61 K 9/22<br>A 61 K 47/00 |
| A | US-A-3 087 819 (R.C. HARRIS)<br>* Spalte 1, Zeilen 26-40, Anspruch 1 * | 1 | |
| A | DE-A-2 852 215 (LOCHER & CO.)<br>* Seite 4, Zeile 1 - Seite 5, Zeile 2, Figur * | 4 | |
| A | DE-A-1 492 881 (W.R. GRACE & CO.)<br>* Seite 19, Absatz 2, Ansprüche 4, 5 * | 1 | |

-----

| RECHERCHIERTE SACHGEBIETE (Int. Cl.4) |
|---|
| A 23 K 1/00<br>A 61 K 9/00<br>A 61 K 47/00 |

Der vorliegende Recherchenbericht wurde für alle Patentansprüche erstellt.

| Recherchenort | Abschlußdatum der Recherche | Prüfer |
|---|---|---|
| BERLIN | 28-11-1986 | SCHULTZE, R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82